# EUROPEAN PATENT APPLICATION

(11) **EP 2 799 443 A1**
(43) Date of publication of application: **05.11.2014**
(21) Application number: 13382161.1
(22) Date of filing: 30.04.2013
(51) Int. Cl.: C07H 21/04, C07D 339/04

(54) **Modified solid support for the synthesis of oligonucleotides**

(71) Applicant: Fundación Imdea Nanociencia, 28049 Madrid (ES)
(72) Inventor: Somoza Calatrava, Álvaro, E-28049 Madrid (ES); Latorre Lozano, Alfonso, E-28049 Madrid (ES)
(74) Representative: ABG Patentes, S.L.

(57) **Abstract**

The present invention refers to a new solid support for the synthesis of oligonucleotides, to a method for its preparation and to its use in the solid phase synthesis of oligonucleotides.

## Description

### Technical Field

The present invention refers to a new solid support for the synthesis of oligonucleotides, to a method for its preparation and to its use in the solid phase synthesis of oligonucleotides.

### Background of the Invention

The applications of oligonucleotides (DNA, RNA and derivatives) in biotechnology and biomedicine are diverse, such as molecular probes to detect specific gene sequences, or to control the gene expression through antisense or RNA interference (RNAi) strategies. One limitation of the use of oligonucleotides in cells or animals is their delivery. For this reason several delivery systems have been evaluated, such as lyposomes, nanotubes and nanoparticles. Among these, gold nanoparticles have shown promising results.

One of the applications of gold nanoparticles and oligonucleotide conjugates is the delivery of small interfering RNAs (siRNAs), which are able to block the expression of specific genes. Particularly, these structures are able to cross the cell membrane and inhibit the target gene. (Giljohann, D. a, Seferos, D. S., Prigodich, A. E., Patel, P. C., & Mirkin, C. a. (2009). Gene regulation with polyvalent siRNA-nanoparticle conjugates. Journal of the American Chemical Society, 131(6), 2072-3).

Gold nanoparticles can be easily modified with oligonucleotides bearing a thiol group, since gold and thiols give rise to strong covalent bonds. The standard preparation of this type of conjugate requires the synthesis of thiol-substituted oligonucleotides by using commercially available modified solid supports which contain a protected thiol group. After oligonucleotide synthesis and purification, the protected thiol group has to be deprotected using an excess of a reagent such as DTT or phosphine derivatives. After incubation, the excess of such reagent is removed using a desalting column and the solution containing the oligonucleotide is concentrated and has to be used in the following hours. Unfortunately, due to the lability of RNA structures, the thiol deprotection step can compromise the integrity of RNA. Moreover, the efficiency of gene inhibition is not good since the release of siRNAs from the nanoparticle is not easy.

The use of a disulfide moiety to connect gold nanoparticles and siRNAs has shown to more efficiently release the siRNAs from the nanoparticles in cells (Lee, J., Green, J. J., Love, K. T., Sunshine, J., Langer, R., & Anderson, D. G. (2009). Gold, Poly(-amino ester) Nanoparticles for Small Interfering RNA Delivery 2009). However, the process disclosed to prepare such conjugates requires several synthetic steps in solution, thus reducing the overall efficiency of the process, and what is more also compromising the integrity of the oligonucleotides. In addition, this approach also requires the previous preparation of the oligonucleotide bearing a protected thiol group, which has to be then deprotected.

In view of the above, the art is still in need of a method that allows the easy preparation of oligonucleotides that can be used for the functionalization of nanoparticles under conditions that do not affect the integrity of the oligonucleotides and which can be easily released from said nanoparticles inside the cells.

### Brief Description of the Invention

The authors of the present invention have developed a new modified solid support which allows the solid phase synthesis of oligonucleotides that can be directly conjugated to metallic surfaces, such as gold or silver nanoparticles, and which are readily released inside the cells. Furthermore, conditions affecting the stability of the oligonucleotides are avoided.

This modified solid support provides a method for the synthesis of oligonucleotides nanoparticle conjugates having the following advantages: (i) oligonucleotides are obtained in good yields, (ii) conditions affecting the stability of the oligonucleotides are avoided, (iii) when the oligonucleotides are obtained, they can be directly reacted with the metallic surface (no thiol deprotection is required), (iv) once inside the cells the oligonucleotide is readily cleaved from the metallic surface under physiological cell conditions.

Accordingly, in one aspect the invention is directed to a modified solid support for oligonucleotide solid phase synthesis having the following formula (I): 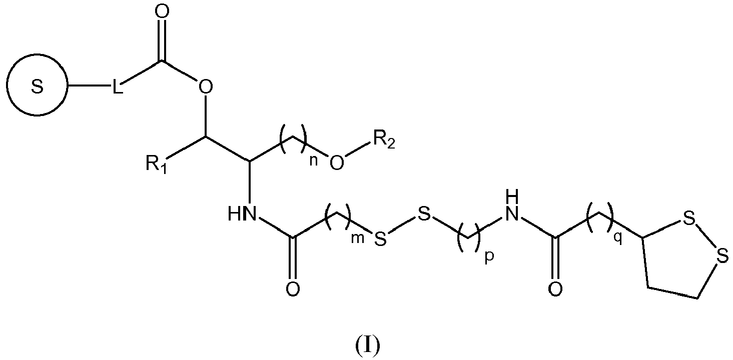 wherein:
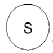 represents a solid support;
L represents a divalent linker;
R₁ is selected from C₁-C₆ alkyl, aryl and heteroaryl;
R₂ is selected from H and a hydroxyl protecting group;
n, m and p are independently an integer selected from 1, 2 and 3; and
q is an integer selected from 2, 3, 4, 5 and 6.

In another aspect, the invention is directed to a method for preparing the modified solid support of the invention comprising:
(i) reacting a compound of formula (II): 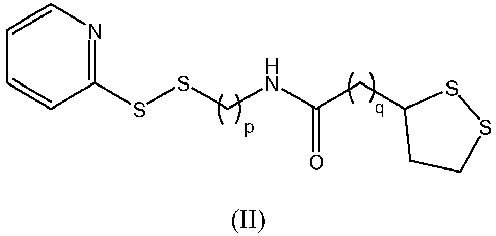 with a thiol of formula (III): 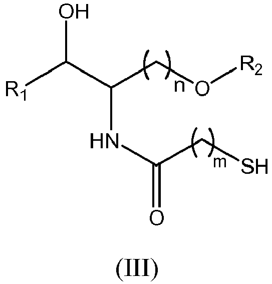 to afford a compound of formula (IV): 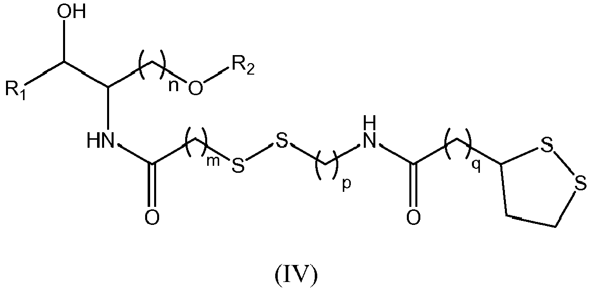
(ii) when R₂ is H, protecting said primary hydroxyl group with a hydroxyl protecting group,
(iii) reacting a compound of formula (IV), wherein R₂ is a hydroxyl protecting group, with a compound of formula (V): 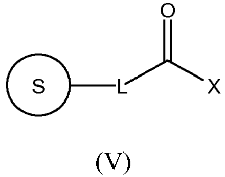
(iv) optionally, deprotecting the hydroxyl protecting group,
wherein:
X is selected from OH, Cl and Br;
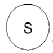 represents a solid support;
L represents a divalent linker;
R₁ is selected from C₁-C₆ alkyl, aryl and heteroaryl;
R₂ is selected from H and a hydroxyl protecting group;
n, m and p are independently an integer selected from 1, 2 and 3; and
q is an integer selected from 2, 3, 4, 5 and 6.

In another aspect, the invention is directed to the use of the modified solid support of the invention in the synthesis of oligonucleotides.

Another aspect of the invention refers to a compound of formula (IV): 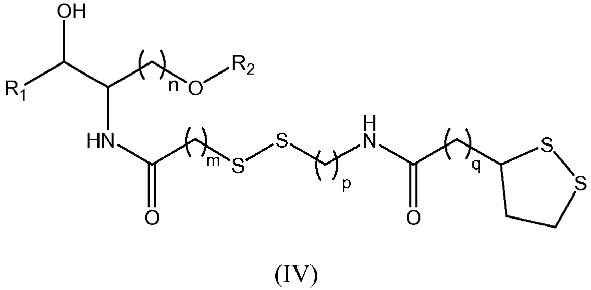 wherein:
R₁ is selected from C₁-C₆ alkyl, aryl and heteroaryl;
R₂ is selected from H and a hydroxyl protecting group;
n, m and p are independently an integer selected from 1, 2 and 3; and
q is an integer selected from 2, 3, 4, 5 and 6.

### Brief description of the drawings

Figure 1 shows the fluorescence spectra of the oligonucleotide duplex bearing a fluorescein molecule released from gold nanoparticles, which have been obtained at different times in an in vitro model when adding: (A) 1 mM of glutathione; (B) 1 µM of glutathione.
Figure 2 shows the fluorescence intensity with a maximum at 517 nm emitted by the oligonucleotide duplex bearing a fluorescein molecule released from gold nanoparticles, which has been obtained at different times in a cell model. Ex: 470, Em: 517 nm.
Figure 3 shows the fluorescence intensity with a maximum at 517 nm emitted by oligonucleotides bearing a fluorescein molecule released from gold nanoparticles.

### Detailed Description of the Invention

In the present invention, the following terms have the meaning as indicated:

The term "oligonucleotide" as used herein refers to an oligomer or polymer of either ribonucleic acid (RNA) or deoxyribonucleic acid (DNA), as well as non-naturally occurring oligonucleotides. Non-naturally occurring oligonucleotides are oligomers or polymers which contain nucleobase sequences which do not occur in nature, or species which contain functional equivalents of naturally occurring nucleobases, sugars, or inter-sugar linkages, like aptamers, spiegelmers, peptide nucleic acids (PNA), threose nucleic acids (TNA), locked nucleic acids (LNA), or glycerol nucleic acids (GNA). This term includes oligomers that contain the naturally occurring nucleic acid nucleobases adenine (A), guanine (G), thymine (T), cytosine (C) and uracil (U), as well as oligomers that contain base analogs or modified nucleobases. Therefore the person skilled in the art understands that the term "oligonucleotide" comprises but is not limited to RNA, DNA and mixed oligonucleotides, antisense oligonucleotides, short interfering RNA (siRNA), microRNAs (miRNAs), aptamers and also spiegelmers. In a particular embodiment, the oligonucleotide is a ribonucleoside (RNA); more particularly it is siRNA.

The term "solid support" refers to conventional solid supports for the synthesis of oligomers, which are well known for the skilled in the art (e.g. Current Protocols in Nucleic Acid Chemistry; Solid-Phase Synthesis: A Practical Guide). The nature of the solid support is not particularly restricted and is within the purview of a person skilled in the art. Thus the solid support may be an inorganic substance. Non-limiting examples of suitable inorganic substances may be selected from the group consisting of silica, porous glass, aluminosilicates, borosilicates, metal oxides and clay containing one or more of these. Alternatively, the solid support may be an organic substance such as a cross-linked polymer. Non-limiting examples of a suitable cross-linked polymer may be selected from the group consisting of polyamide, polyether, polystyrene and mixtures thereof. The preferred solid support for use herein is conventional and may be selected from controlled pore glass (CPG) and polystyrene beads.

The linker L in the modified solid support can be a wide variety of handles used in solid phase oligonucleotide synthesis. The linker L is a bifunctional spacer that, on one end, incorporates a functional group, often a carboxyl group, that allows coupling to the rest of the molecule through an ester group, and on the other end, a functional group that allows coupling to the solid support. In a particular embodiment, the linker L is an alkyl chain bounded to the solid support through an amide group and to the rest of the molecule through an ester group.

The term "hydroxyl protecting group" as used herein refers to those groups which are intended to protect a hydroxyl group against undesirable reactions during synthetic procedures. Hydroxyl protecting groups are well known in the art (e.g. T. W. Greene, P. G. M. Nuts, "Protecting Groups in Organic Synthesis", Second Edition (1991), John Wiley and Sons, Inc., New York). Non-limiting examples of useful protecting groups may be selected from the group consisting of trityl, methoxytrityl, dimethoxytrityl (DMT), dialkylphosphite, pivalyl-isobutyloxycarbonyl, t-butyldimethylsilyl, phenoxyacetal, 9-phenylxanthen-9-yl (pixyl), tetrahydropyranyl, methoxytetrahydropyranyl, methoxymethyl, benzyloxymethyl, methoxyethoxymethyl, methylthiomethyl, dialkylphosphate, levulinyl, dimethylphenylsilyl, trimethylsilyl, isopropyldimethylsilyl, diisopropylmethylsilyl, diethylisopropylsilyl, triisopropylsilyl, acetyl, benzoyl, pivaloyl, trifluoroacetyl, allyl, benzyl, o-nitrobenzyl, o-hydroxystyryldimethylsilyl, 2-oxo-1,2-diphenylethyl, allyloxycarbonyl, monomethoxymethyl, nitroveratryloxycarbonyl, dimethoxybenzoin, dimethoxybenzoin carbonate, methylnitropiperonyl carbonate, fluorenylmethoxycarbonyl, 2-phenylsulfonylethoxycarbony, fluorophenylmethoxypiperidinyl and the like. In a particular embodiment, the hydroxyl protecting group is dimethoxytrityl (DMT).

The term "C₁-C₆ alkyl" as used herein refers to a linear or branched alkyl group containing from 1 to 6, preferably from 1 to 3 (C₁-C₃ alkyl), carbon atoms; examples of such groups include methyl, ethyl, propyl, isopropyl, n-butyl, isobutyl, tert-butyl, pentyl or hexyl. Preferably, it is methyl.

An "aryl" group refers to an all-carbon monocyclic or fused-ring polycycle group having a completely conjugates pi-electron system. Non-limiting examples of aryl groups are phenyl, naphthalenyl and anthracenyl.

A "heteroaryl" group refers to a monocyclic or fused ring group having in the ring(s) one or more atoms selected from nitrogen, oxygen and sulfur and, in addition, having a completely conjugated pi-electron system. Non-limiting examples of heteroaryl groups are pyrrole, furan, thiophene, imidazole, oxazole, thiazole, pyrazole, pyridine, pyrimidine, quinoline, isoquinoloine, purine and carbazole.

As mentioned before, a first aspect of the present invention is directed to a modified solid support for oligonucleotide solid phase synthesis having the following formula: 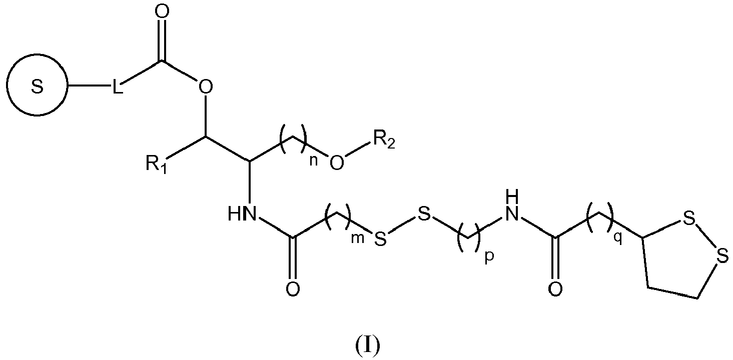 wherein:
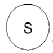 represents a solid support;
L represents a divalent linker;
R₁ is selected from C₁-C₆ alkyl, aryl and heteroaryl;
R₂ is selected from H and a hydroxyl protecting group;
n, m and p are independently an integer selected from 1, 2 and 3; and
q is an integer selected from 2, 3, 4, 5 and 6.

The inventors have identified the following moieties as key features in the modified solid support of the invention:
- a primary hydroxyl group used as the foundation on which the oligonucleotide is synthesized and which can be masked with a protecting group;
- an ester group that allows attachment to the solid support and that can be further cleaved to detach the oligonucleotide from the solid support,
- a dithiolane moiety at one end that allows direct binding to metallic surfaces, such as gold or silver nanoparticles that are used to deliver the oligonucleotide to the cells, and
- a disulfide group connecting the dithiolane moiety at one end of the molecule and the hydroxyl group used to grow the oligonucleotide. This disulfide group is cleaved inside the cells thus releasing the oligonucleotide.

In a particular embodiment, R₁ is selected from C₁-C₆ alkyl and phenyl. Preferably, R₁ is selected from C₁-C₃ alkyl and phenyl, more preferably R₁ is a C₁-C₃ alkyl group, even more preferably R₁ is methyl.

In another particular embodiment, R₂ is selected from H, trityl, methoxytrityl, dimethoxytrityl, dialkylphosphite, pivalyl-isobutyloxycarbonyl, t-butyldimethylsilyl, phenoxyacetal, 9-phenylxanthen-9-yl, tetrahydropyranyl, methoxytetrahydropyranyl, methoxymethyl, benzyloxymethyl, methoxyethoxymethyl, methylthiomethyl, dialkylphosphate, levulinyl, dimethylphenylsilyl, trimethylsilyl, isopropyldimethylsilyl, diisopropylmethylsilyl, diethylisopropylsilyl, triisopropylsilyl, acetyl, benzoyl, pivaloyl, trifluoroacetyl, allyl, benzyl, o-nitrobenzyl, o-hydroxystyryldimethylsilyl, 2-oxo-1,2-diphenylethyl, allyloxycarbonyl, monomethoxymethyl, nitroveratryloxycarbonyl, dimethoxybenzoin, dimethoxybenzoin carbonate, methylnitropiperonyl carbonate, fluorenylmethoxycarbonyl, 2-phenylsulfonylethoxycarbony, fluorophenylmethoxypiperidinyl. Preferably, R₂ is selected from H, trityl, methoxytrityl, dimethoxytrityl. More preferably, it is dimethoxytrityl (DMT).

In another particular embodiment, n is 1.

In another particular embodiment, m is 2.

In another particular embodiment, p is 2.

In another particular embodiment, q is 4.

In a preferred embodiment of the invention, R₁ is methyl, n is 1, m and p are 2 and q is 4.

In another particular embodiment, the solid support is selected from inorganic substances, such as silica, porous glass, aluminosilicates, borosilicates, metal oxides and clay containing one or more of these, and organic substances, such as polyamide, polyether, polystyrene and mixtures thereof. In a particular embodiment, the solid support is selected from controlled pore glass (CPG) and polystyrene beads. More particularly, the solid support is CPG.

In another particular embodiment of the present invention, the linker L has the following formula: 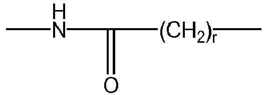 wherein r is an integer selected from 1, 2, 3, 4, 5 and 6, preferably 2.

In another preferred embodiment, the modified solid support of the present invention has the following formula: 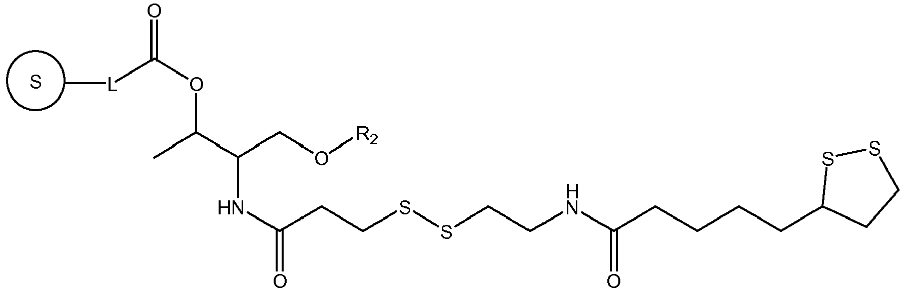 wherein:
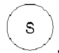 L and R₂ are as defined above.

The organic structure of the modified solid support of the invention represented by the above described formula (I) may include enantiomers, diastereoisomers or mixtures thereof.

In a particular embodiment, the modified solid support has the following formula: 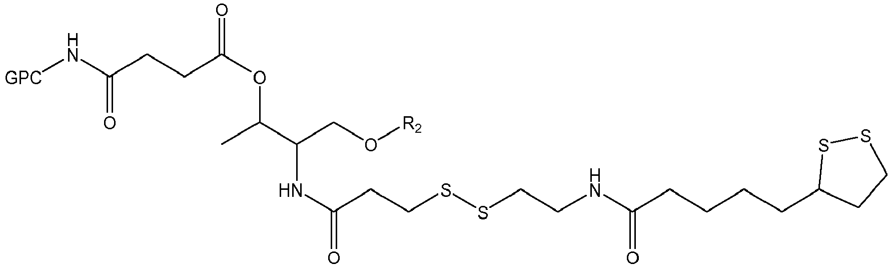 wherein R₂ is as defined above.

In a preferred embodiment, the modified solid support has the following formula: 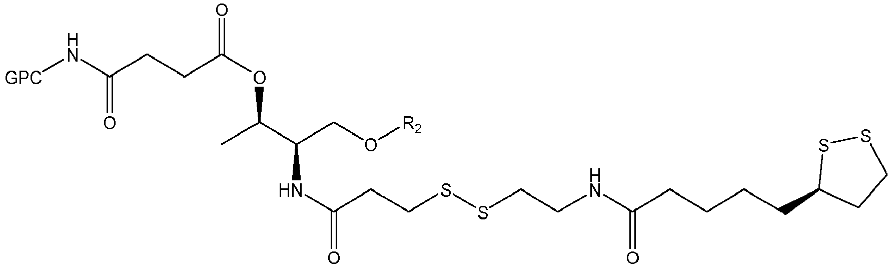 wherein R₂ is as defined above; or an enantiomer, diastereoisomer or mixtures thereof. Preferably, R₂ is selected from H, trityl, methoxytrityl, dimethoxytrityl. More preferably, it is dimethoxytrityl (DMT).

In another aspect, the invention refers to a method for the preparation of the modified solid support of the invention. Said method comprises:
(i) reacting a compound of formula (II): 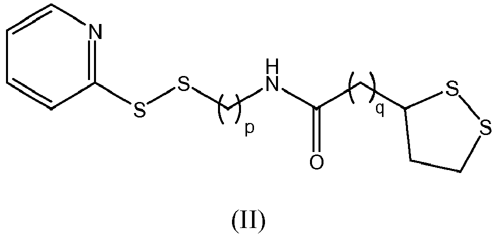 with a thiol of formula (III): 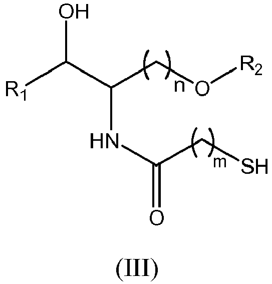 to afford a compound of formula (IV): 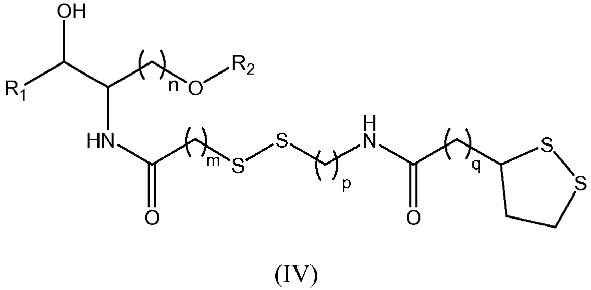
(ii) when R₂ is H, protecting said primary hydroxyl group with a hydroxyl protecting group,
(iii) reacting a compound of formula (IV), wherein R₂ is a hydroxyl protecting group, with a compound of formula (V): 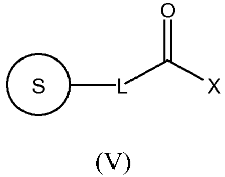
(iv) optionally, deprotecting the hydroxyl protecting group,
wherein:
X is selected from OH, Cl and Br;
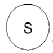 represents a solid support;
L represents a divalent linker;
R₁ is selected from C₁-C₆ alkyl, aryl and heteroaryl;
R₂ is selected from H and a hydroxyl protecting group;
n, m and p are independently an integer selected from 1, 2 and 3; and
q is an integer selected from 2, 3, 4, 5 and 6.

The above chemical transformations can be carried out using conventional synthetic methods and reactions conditions known in the art for such type of transformations, such as those described in the examples of the present invention.

The inventors have observed that the modified solid support of the invention is a reagent suitable for use in a DNA synthesizer for the solid phase preparation of oligonucleotides. When R₂ is a hydroxyl protecting group, it may be directly deprotected in the DNA synthesizer. In this way, the oligonucleotide (ON) was synthesized on the primary hydroxyl group affording compound (VI): 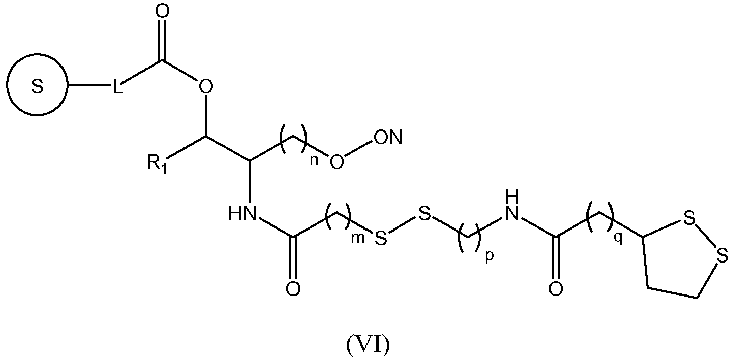 wherein:
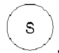 L, R₁ n, m, p and q are as defined above.

Once the desired oligonucleotide was prepared using standard automated oligonucleotide synthesis methods, the solid support was cleaved giving rise to the oligonucleotide (VII) which is functionalized with the chemical modification of the invention: 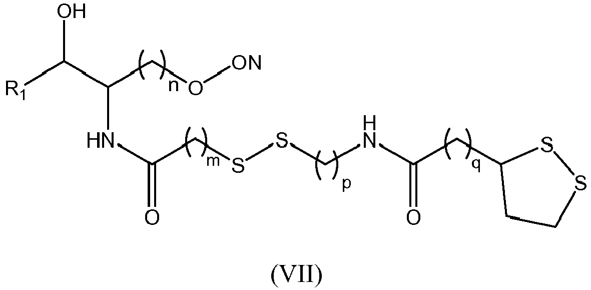 wherein:
R₁ n, m, p and q are as defined above.

This oligonucleotide may be directly attached to metallic surfaces, such as gold nanoparticles, just by treating the oligonucleotide (VII) with the metallic surface to afford an oligonucleotide nanoparticle conjugated (VIII), thus avoiding the need of a thiol deprotection step, as in the methods of the prior art, which could compromise the stability of the oligonucleotide. 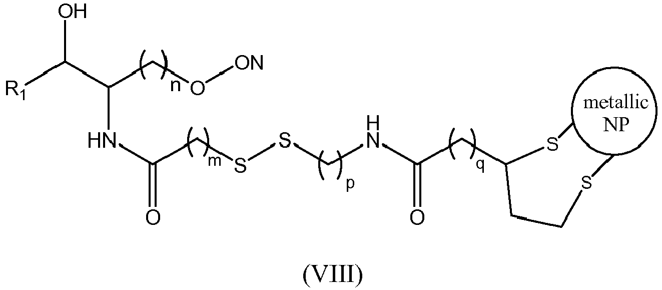 wherein:
R₁ n, m, p and q are as defined above.

In a particular embodiment, R₁ is selected from C₁-C₆ alkyl and phenyl. Preferably, R₁ is selected from C₁-C₃ alkyl and phenyl, more preferably R₁ is a C₁-C₃ alkyl group, even more preferably R₁ is methyl.

In another particular embodiment, n, m and p are independently an integer selected from 1, 2 and 3. In a preferred embodiment, n is 1. In another preferred embodiment, m is 2. In another preferred embodiment, p is 2.

In another particular embodiment, q is an integer selected from 2, 3, 4, 5 and 6. Preferably, q is 4.

In another preferred embodiment, in the oligonucleotide nanoparticle conjugated (VIII) R₁ is methyl, n is 1, m is 2, p is 2 and q is 4.

Even more preferably, the oligonucleotide nanoparticle conjugated (VIII) is:

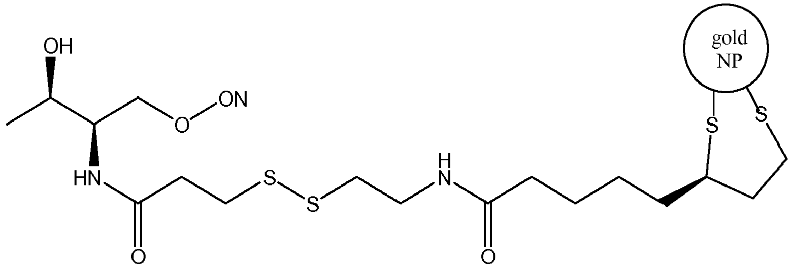

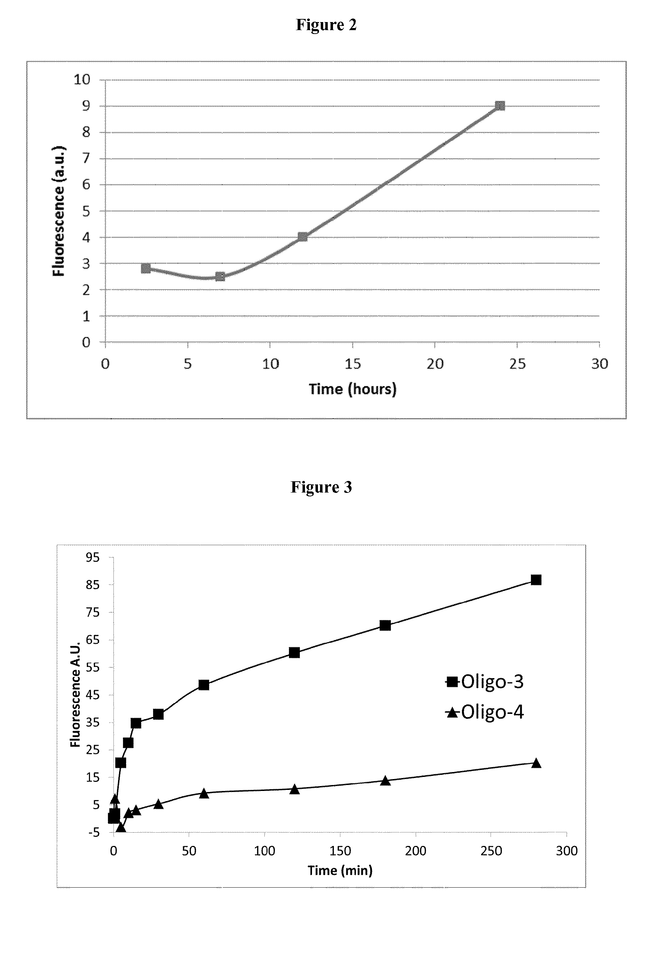

The resulting oligonucleotide nanoparticle conjugates (VIII) are efficiently delivered to cells and, once inside the cells, the oligonucleotide (IX) was readily released under physiological conditions inside the cells (see Example 11 and Figure 2). 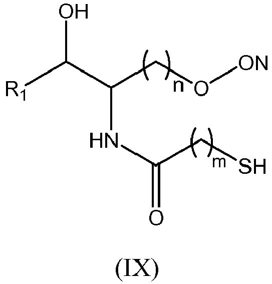 wherein:
R₁ n and m are as defined above.

In a particular embodiment, R¹ is selected from C₁-C₆ alkyl and phenyl. Preferably, R¹ is selected from C₁-C₃ alkyl and phenyl, more preferably R¹ is a C₁-C₃ alkyl group, even more preferably R¹ is methyl.

In another particular embodiment, n and m are independently an integer selected from 1, 2 and 3. In a preferred embodiment, n is 1. In another preferred embodiment, m is 2.

In another preferred embodiment, in the oligonucleotide (IX) R¹ is methyl, n is 1 and m is 2.

Even more preferably, the oligonucleotide (IX) is:

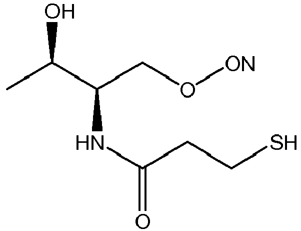

In view of the above, the modified solid support of the present invention allows a method for the synthesis of oligonucleotides than can be readily utilized in the preparation of metallic surfaces, such as nanoparticles, functionalized with oligonucleotides, which can be further released under very mild conditions.

In particular, this method has several advantages over the processes known in the prior art, such as:
- oligonucleotides functionalized with the required chemical modification for their further attachment to the metallic surface can be directly obtained in good yields using standard automated oligonucleotides synthesis methods;
- the resulting oligonucleotides can be easily cleaved from the solid support;
- the oligonucleotides obtained after solid phase synthesis can be directly attached to the metallic surface;
- the resulting metallic surfaces functionalized with the oligonucleotide are efficiently delivered to cells;
- the oligonucleotide is readily cleaved from the metallic surface under physiological conditions inside the cells;
- this strategy provides a very short and direct synthesis of the oligonucleotide functionalized metallic surface (solid phase synthesis of the oligonucleotide/cleavage from the solid support/attachment to the metallic surface), thus avoiding or minimizing the possible degradation of the oligonucleotide caused by its manipulation;
- the whole method can be performed under very mild conditions thus not affecting the stability of the oligonucleotide;
- the modified solid support of the invention is a very stable compound that can be prepared independent of the oligonucleotide synthesis and that is a reagent suitable for use in a DNA synthesizer. Accordingly, the modified solid support of the invention can be previously prepared in large scale and store for its further use in the preparation of numerous different oligonucleotides.

Therefore, in another aspect, the invention refers to a method for the synthesis of oligonucleotides comprising the use of a modified solid support as defined above. This method may be carried out according to the procedure described herein before.

Another object of the present invention is an intermediate useful in the preparation of the modified solid support of the present invention. Accordingly, in another aspect, the invention refers to a compound of formula (IV): 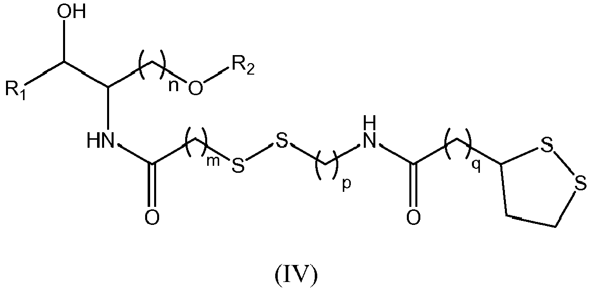 wherein:
R¹ is selected from C₁-C₆ alkyl, aryl and heteroaryl;
R² is selected from H and a hydroxyl protecting group;
n, m and p are independently an integer selected from 1, 2 and 3; and
q is an integer selected from 2, 3, 4, 5 and 6.

In a particular embodiment, R¹ is selected from C₁-C₆ alkyl and phenyl. Preferably, R¹ is selected from C₁-C₃ alkyl and phenyl, more preferably R¹ is a C₁-C₃ alkyl group, even more preferably R¹ is methyl.

In another particular embodiment, n is 1.

In another particular embodiment, m is 2.

In another particular embodiment, p is 2.

In another particular embodiment, q is 4.

In a preferred embodiment, in the compound of formula (IV) R¹ is methyl, n is 1, m is 2, p is 2 and q is 4.

In an embodiment, the compound of formula (IV) is a compound of the following formula: 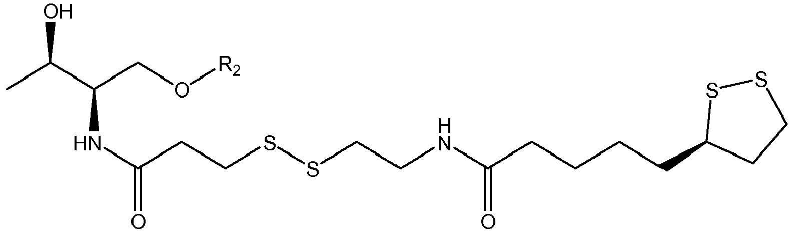 wherein R² is as defined above; or an enantiomer, diastereoisomer or mixtures thereof. Preferably, R² is selected from H, trityl, methoxytrityl, dimethoxytrityl. More preferably, it is dimethoxytrityl (DMT).

The invention will be further described by reference to the following detailed examples. These examples are offered to further illustrate the various specific and illustrative embodiments and techniques. It should be understood however that variations and modifications may be made while remaining within the scope of the present invention.

### Examples

### Example 1: Synthesis of N-((2R,3R)-1,3-dihydroxybutan-2-yl)-3-mercaptopropanamide (A).

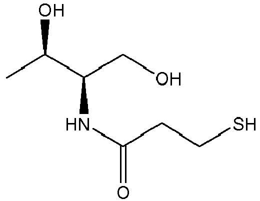

To a stirred mixture of 3-mercaptopropionic acid (100 mg, 0.94 mmol), N-hydroxybenzotriazole (135 mg, 1 mmol) and N,N'-Dicyclohexylcarbodiimide (206 mg, 1 mmol) in DMF (3 mL) under N₂, L-threoninol (100 mg, 0.94 mmol) was added at room temperature. After 16 h the reaction mixture was quenched with MeOH and the solvent evaporated in vacuum. To the residue 30 mL of CH₂Cl₂ was added, and the solid filtered off. After solvent evaporation and flash chromatography (eluent CH₂Cl₂/MeOH 15:1) compound A was obtained as a colorless oil, in 65% yield; ¹H NMR (300 MHz, CDCl₃) δ 6.55 (s, 1H), 4.17 (qd, J = 6.1, 2.0 Hz, 1H), 4.03 - 3.64 (m, 3H), 3.02 - 2.67 (m, 2H), 2.57 (t, J = 6.6 Hz, 2H), 1.63 (t, J = 8.3 Hz, 1H), 1.20 (d, J = 6.4 Hz, 3H); ¹³C NMR (75MHz, CDCl₃) δ172.2, 67.7, 63.9, 55.1, 40.3, 29.6, 20.5; MS (ESI):m/z (%) 176 (M⁺-OH, 11), 194 (M⁺+1, 10), 216 (M⁺+Na, 100); HRMS (ESI) calcd for C₇H₁₅NO₃S (M⁺+1) 216.0660, found 216.0664.

### Example 2: Synthesis of 2-(Pyridyldithio)-ethylaminehydrochloride(PDA*HCl).

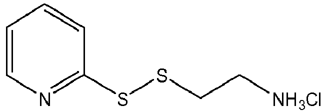

PDA*HCl was synthesized as reported (G.T. Zugates, D. G. Anderson, S. R. Little, I. E. B. Lawhorn, R Langer, J. Am. Chem. Soc. 2006, 128, 12726-12734 ) with some modifications. To a stirred solution of aldrithiol (213 mg, 0.96 mmol) in MeOH (1.1 mL), 2-mercaptoethylamine hydrochloride (109 mg, 0.96 mmol) was added. After stirring 1h, the solvent was evaporated and the residue washed with cold AcOEt three times. PDA*HCl was obtained as a white solid in 51% yield: ¹H NMR (300 MHz, d⁶-CD₃OD) δ8.57 (d, J = 5.0 Hz, 1H), 7.83 (t, J = 7.7 Hz, 1H), 7.69 (d, J = 8.0 Hz, 1H), 7.35 (dd, J = 7.5, 5.0 Hz, 1H), 3.18 (t, J = 6.1 Hz, 2H), 3.07 (t, J= 6.8 Hz, 2H); MS (ESI):m/z (%) 107 (100), 153 (79), 187 (M⁺-Cl, 12); HRMS (ESI) calcd for C₇H₁₁NS₂(M⁺+1) 187.0366, found 187.0391.

### Example 3: Synthesis of (R)-5-(1,2-dithiolan-3-yl)-N-(2-(pyridin-2-yldisulfanyl)ethyl)pentanamide (B).

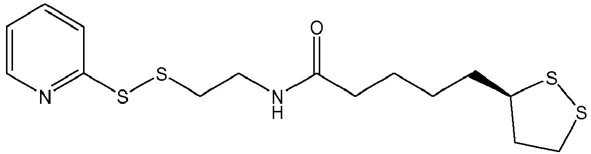

To a stirred mixture of (R)-(+)-α-Lipoic acid (134mg, 0.65 mmol), N-hydroxybenzotriazole (96 mg, 0.71 mmol) and N,N'-Dicyclohexylcarbodiimide (147, 0.71 mmol) in DMF (1.7 mL) under N₂, PDA*HCl (145 mg, 0.65 mmol) and DIPEA (147 µL, 0.71 mmol) were added at room temperature. After 16 h the reaction mixture was quenched with MeOH and the solvent evaporated in vacuum. To the residue 30 mL of CH₂Cl₂ was added, and the solid filtered off. After solvent evaporation and flash chromatography (eluent CH₂Cl₂/AcOEt 1:1) compound B was obtained as a colorless oil, in 14% yield; ¹H NMR (300 MHz, CDCl₃) δ 8.48 (dd, J = 3.7, 1.1 Hz, 1H), 7.70 - 7.56 (m, 1H),7.51 (d, J = 8.0 Hz, 1H), 7.23 - 7.08 (m, 2H), 3.64 - 3.46 (m, 3H), 3.22 - 3.02 (m, 2H), 2.96 - 2.85 (m, 2H), 2.43 (dq, J = 12.5, 6.4 Hz, 1H), 2.21 (t, J = 7.4 Hz, 2H), 1.97 - 1.80 (m, 1H), 1.78 - 1.57 (m, 5H), 1.54 - 1.38 (m, 2H); ¹³C NMR (75MHz, CDCl₃) δ172.9, 159.2, 149.7, 137.1, 121.4, 121.2, 95.7, 77.5, 77.1, 76.7, 56.4, 40.3, 39.0, 38.5, 37.3, 36.6, 34.7, 29.0, 25.4; MS (ESI):m/z (%) 225 (52), 264 (10), 375 (M⁺+1, 100); HRMS (ESI) calcd for C₁₅H₂₃N₂S₄(M⁺+1) 375.0675, found 375.0687.

### Example 4: Synthesis of N-(2-((3-((2R,3R)-1,3-dihydroxybutan-2-ylamino)-3-oxopropyl)disulfanyl)ethyl)-5-((R)-1,2-dithiolan-3-yl)pentanamide (C).

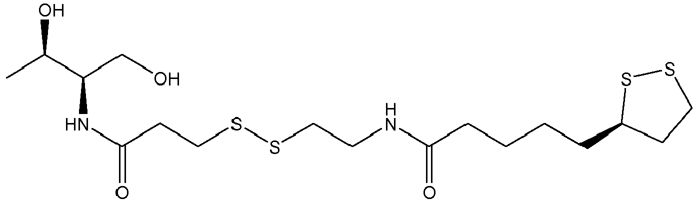

To a solution of disulfide B (45 mg, 0.12 mmol) in MeOH (1mL) under N₂, a solution of compound A (23mg, 0.12 mmol) in MeOH (1 mL) was added and stirred for 2 h. The solvent was evaporated and the residue purified by flash chromatography (CH₂Cl₂/MeOH 20:1) to obtain compound C as colorless oil in 88% yield; ¹H NMR (300 MHz, CDCl₃) δ6.88 (d, J = 8.3 Hz, 1H), 6.61 (t, J = 5.6 Hz, 1H), 4.23 - 4.03 (m, 1H), 3.91-3.74 (m, 5H), 3.62 - 3.47 (m, 3H), 3.02 (t, J = 6.8 Hz, 2H), 2.83 (t, J = 6.5 Hz, 2H), 2.68 (t, J = 6.7 Hz, 2H), 2.45 (dq, J = 12.4, 6.4 Hz, 1H), 2.22 (t, J = 7.4 Hz, 2H), 1.90 (dq, J = 13.7, 6.9 Hz, 1H), 1.78 - 1.55 (m, 5H), 1.45 (m, 3H), 1.19 (d, J = 6.4 Hz, 3H).13C NMR (75 MHz, CDC13) δ 173.9, 171.9, 68.6, 64.6, 56.4, 55.1, 40.2, 38.6, 38.5, 38.3, 36.5, 36.3, 34.9, 34.5, 28.8, 25.3, 20.4; MS (ESI): m/z (%) 301 (13), 457 (M⁺+1, 14), 479 (M⁺+Na, 100); HRMS (ESI) calcd for C₁₇H₃₃N₂O₄S₄(M⁺+1) 457.1334, found 457.1317; HRMS (ESI) calcd for C₁₇H₃₂N₂O₄NaS₄ (M+) 479.1166, found 479.1137.

### Example 5: Synthesis of N-(2-((3-((2R3R)-1-(bis(4-methoxyphenyl)(phenyl)methoxy)-3-hydroxybutan-2-ylamino)-3-oxopropyl)disulfanyl)ethyl)-5 -((R)-1,2-dithiolan-3-yl)pentanamide (D).

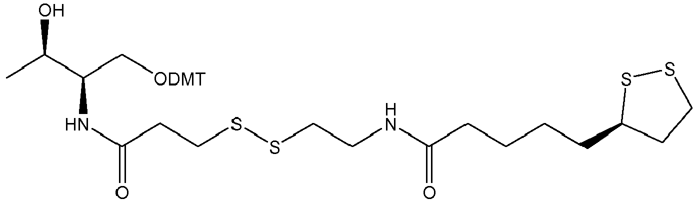

To a solution of compound C (74 mg, 0.16 mmol) in pyridine (0.8 mL) at 0 °C, DIPEA (43 µL, 0.24 mmol) and 4,4'-dimethoxytrithylchloride ( 64 mg, 0.19 mmol) were added. The mixture was stirred and allowed to reach room temperature slowly. After 16 h, the solvent was evaporated and the residue purified by flash chromatography (eluent Hex/AcOEt 1:9 using silica gel deactivated with Et₃N) to obtain compound D in 35% yield as a yellow solid; ¹H NMR (300 MHz, CDC13) δ7.39 - 7.27 (m, 2H), 7.27 - 7.11 (m, 7H), 6.76 (d, J = 8.8 Hz, 4H), 6.41 - 6.21 (m, 2H), 4.13 - 3.96 (m, 2H), 3.95 - 3.83 (m, 1H), 3.71 (s, 6H), 3.42-3.51 (m, 3H), 3.28 (ddd, J = 36.7, 9.6, 4.1 Hz, 2H), 3.13 - 2.97 (m, 2H), 2.92 (t, J = 6.8 Hz, 2H), 2.73 (t, J = 6.2 Hz, 2H), 2.57 (td, J = 6.9, 2.7 Hz, 2H), 2.45 - 2.26 (m, 1H), 2.10 (t, J = 7.4 Hz, 2H), 1.80 (dq, J = 13.5, 6.9 Hz, 1H), 1.57 (qd, J = 13.2, 11.1, 6.8 Hz, 4H), 1.46 - 1.26 (m, 2H), 1.07 (d, J = 6.4 Hz, 3H); MS (ESI): m/z (%) 303 (100), 781 (M⁺+Na, 15); HRMS (ESI) calcd for C₃₈H₅₀N₂O₆S₄ (M⁺+Na) 781.2455, found 781.2443.

### Example 6: Synthesis of Solid Support (CPG) Preparation.

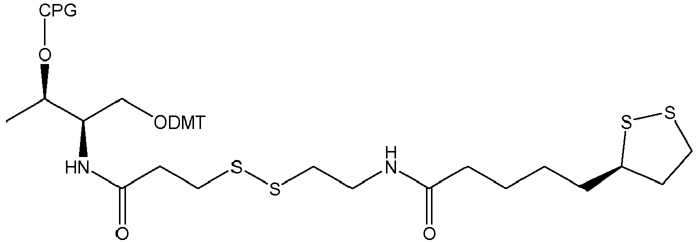

To a solution of compound D (40 mg, 0.052mmol) in CH₂Cl₂(0.4 mL), succinic anhydride (6.8 mg, 0.068 mmol), DIPEA (13 µL, 0.072 mmol) and DMAP (catalytic amount) were added under N₂ at room temperature. The mixture was stirred during 16 h, washed with water and dried with MgSO₄. After solvent evaporation, the residue obtained was dissolved in DMF (1.2 mL), and HBOt (7 mg, 0.052 mmol) and DCC (10 mg, 0.052 mmol) were added. This mixture was added to 400 mg of CPG (500 Å) and stirred during 2h. The solvent was removed and the CPG washed with CH₂Cl₂ gently. Once the CPG was dry, 2 mL of a 1:1 mixture of capping reagents used on oligonucleotide synthesis [CAP MIX A: Acetic anhydride (400 µL)/ Py (600 µL)/ THF (500 µL); CAP MIX B: 1-Methylimidazol (400 µL)/ THF (1 mL)] was added and stirred. After 25 min, the modified CPG was washed with MeOH, CH₃CN, and dried.

### Example 7: Oligonucleotide synthesis.

The modified oligonucleotide was prepared using a DNA Synthesizer (MerMade4) using the standard parameters. The solid support described in Example 6 was used in the preparation of the oligonucleotide (Oligo-1).

Oligonucleotide thus obtained was deprotected using standard procedures (concentrated ammonia 2h, at 55 C). Purification was done by polyacrylamide gel electrophoresis, giving rise to compound Oligo-1: 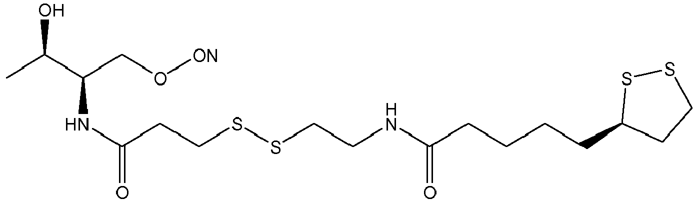 wherein ON is the sequence: 5'-TCGAAGTATTCCGCGTACGTTTTTTT-3' (SEQ ID NO: 1), bound through the 3' position.

Standard preparation and purification procedures can be found e.g. in: Current Protocols in Nucleic Acid Chemistry.

Fluorescein modified Oligo-2, having the sequence: CGTACGCGGAATACTTCGAT (SEQ ID NO: 2), was prepared using commercially available solid support (3'-fluorescein CPG, Link Technologies) as described above, followed by deprotection and purification. The oligonucleotide is bound to fluorescein at position 3'.

Oligo-3, having the sequence: TTTTTTTTTT (SEQ ID NO: 3), was prepared using the solid support as in Oligo-1 and a commercially available phosphoramidite (5'-Fluorescein-CE Phosphoramidite, Link Technologies), followed by deprotection and purification. The oligonucleotide is bound to fluorescein at position 5' and the modification herein reported at position 3'

Oligo-4, having the sequence: TTTTTTTTTT (SEQ ID NO: 3), was prepared using a solid support having the dithiolane moiety but not the disulfide group and a commercially available phosphoramidite (5'-Fluorescein-CE Phosphoramidite, Link Technologies), followed by deprotection and purification. The oligonucleotide is bound to fluorescein at position 5' and the dithiolane group position 3'

### Example 8: Preparation of Oligonucleotide Duplex.

The modified oligonucleotides prepared in example 7 (Oligo-1 and Oligo-2) were annealed by mixing both oligonucleotides in a 1:1 ratio in buffer , heating the solution at 90°C and cooling down slowly the mixture to obtain the corresponding oligonucleotide duplex.

### Example 9: Functionalization of nanoparticles

Gold nanoparticles were incubated overnight with the oligonucleotide duplex (750 equiv.) prepared in example 8 and the Oligo-3 and Oligo-4 prepared in example 7 in the presence of NaCl (0.3 M). The three samples were purified by centrifugation, the supernatants were removed and the particles dissolved in water. This process was repeated 3 times.

### Example 10: In vitro evaluation of modified nanoparticles

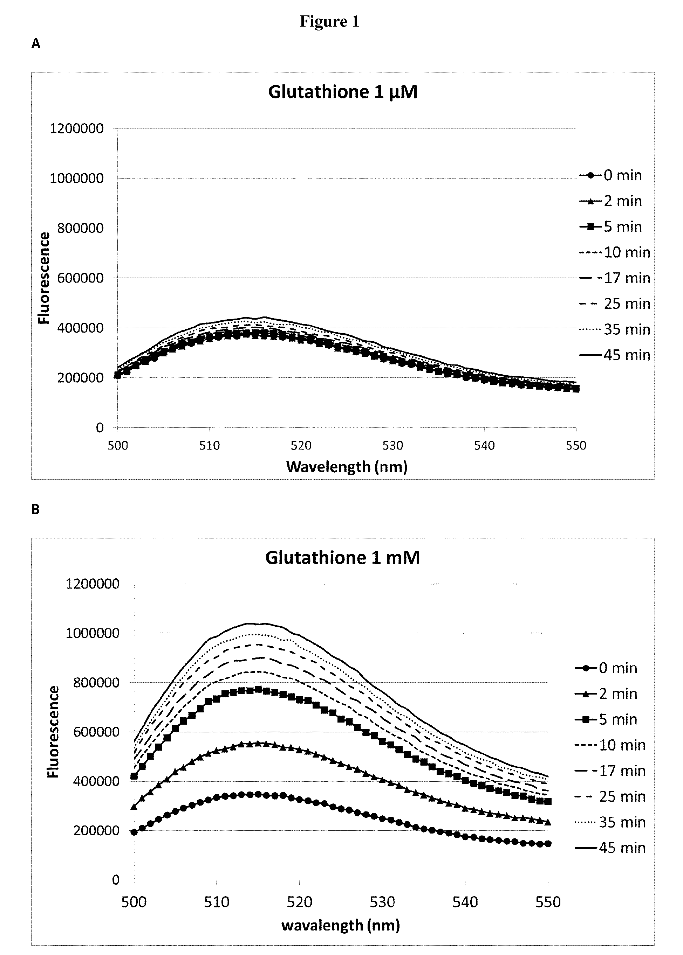

To a solution of modified gold nanoparticles prepared in Example 9 with the oligonucleotide duplex in PBS was added glutathione (final concentration 1 µM or 1 mM). The *in vitro* release of the oligonucleotide duplex bearing the fluorescein molecule is followed by fluorescence. The fluorescence was recorded at different periods of time. The results showed that the release is promoted by the addition of glutathione at high concentration (Fig 1B), such that one found inside cells (1 mM), but the release is very slow (Fig 1A) at concentrations found outside of the cells (1 µM). The fluorescence is quenched when fluorescein is close to the gold nanoparticle, but it increases over time due to the release of the fluorescent oligonucleotide promoted by glutathione.

### Example 11: Evaluation of the release system in cells.

Human breast cancer cells (MCF-7) were maintained in RPMI 1640 media supplemented with 10% FBS and grown at 37°C under 5% CO₂ until they reached 80-90% confluency.

Cells were incubated with modified nanoparticles prepared in Example 9 and the fluorescence recorded every 6 hours (Figure 2). The fluorescence increases overtime pointing out that the modified gold nanoparticles are able to translocate inside the cell and release the oligonucleotide duplex therein.

### Example 12: Comparative release of Oligo-3 and Oligo-4

To a solution of modified gold nanoparticles prepared in Example 9 in PBS bearing the Oligo-3 or Oligo-4 was added glutathione (final concentration 1 mM). The *in vitro* release of the oligonucleotides bearing the fluorescein molecule is followed by fluorescence. The fluorescence was recorded at different periods of time. The results showed that the release takes place efficiently in the case of Oligo-3 but not in the case of Oligo-4 (Figure 3). Particularly, in the case of Oligo-3, the fluorescence increases fast due to the release of the oligonucleotide from the gold nanoparticle in the presence of glutathione at 1 mM. The fluorescence is quenched when fluorescein is close to the gold nanoparticle, but it increases over time due to the release of the fluorescent oligonucleotide in the presence of glutathione. In the case of the Oligo-4 (comparative experiment), where the oligonucleotide contains only the dithiolane moiety required to bind the gold nanoparticle but lacks the disulfide group, the fluorescence observed is low due to the inefficient release in the presence of glutathione at 1 mM. Ex: 470, Em: 517 nm.

## Claims

1. A modified solid support for oligonucleotide solid phase synthesis having the following formula (I): 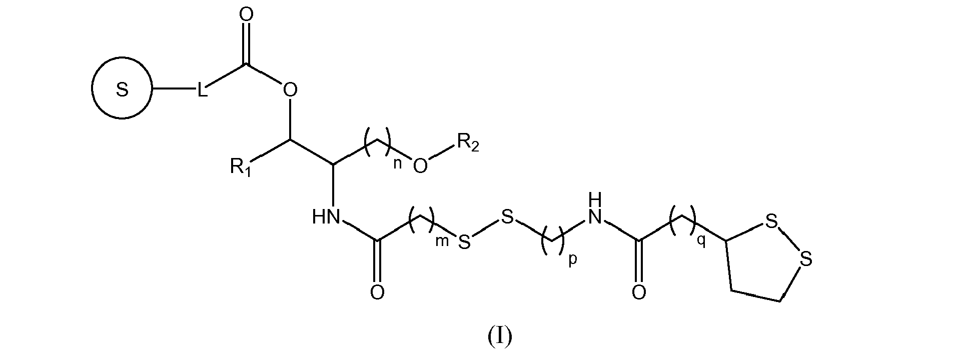 wherein:
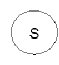 represents a solid support;
L represents a divalent linker;
R₁ is selected from C₁-C₆ alkyl, aryl and heteroaryl;
R₂ is selected from H and a hydroxyl protecting group;
n, m and p are independently an integer selected from 1, 2 and 3; and
q is an integer selected from 2, 3, 4, 5 and 6.

2. A modified solid support according to claim 1, wherein R₂ is selected from H, trityl, methoxytrityl, dimethoxytrityl, dialkylphosphite, pivalyl-isobutyloxycarbonyl, t-butyldimethylsilyl, phenoxyacetal, 9-phenylxanthen-9-yl, tetrahydropyranyl, methoxytetrahydropyranyl, methoxymethyl, benzyloxymethyl, methoxyethoxymethyl, methylthiomethyl, dialkylphosphate, levulinyl, dimethylphenylsilyl, trimethylsilyl, isopropyldimethylsilyl, diisopropylmethylsilyl, diethylisopropylsilyl, triisopropylsilyl, acetyl, benzoyl, pivaloyl, trifluoroacetyl, allyl, benzyl, o-nitrobenzyl, o-hydroxystyryldimethylsilyl, 2-oxo-1,2-diphenylethyl, allyloxycarbonyl, monomethoxymethyl, nitroveratryloxycarbonyl, dimethoxybenzoin, dimethoxybenzoin carbonate, methylnitropiperonyl carbonate, fluorenylmethoxycarbonyl, 2-phenylsulfonylethoxycarbonyl, fluorophenylmethoxypiperidinyl.

3. A modified solid support according to any one of claims 1 and 2, wherein R₁ is selected from C₁-C₃ alkyl and phenyl.

4. A modified solid support according to any one of claims 1 to 3, wherein n is 1.

5. A modified solid support according to any one of claims 1 to 4, wherein m is 2.

6. A modified solid support according to any one of claims 1 to 5, wherein p is 2.

7. A modified solid support according to any one of claims 1 to 6, wherein q is 4.

8. A modified solid support according to any one of claims 1 to 7, wherein the solid support is selected from controlled pore glass (CPG) and polystyrene beads.

9. A modified solid support according to any one of claims 1 to 8, wherein the linker L has the following formula: 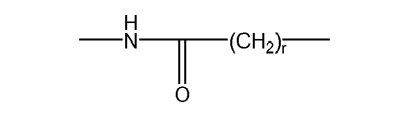 wherein r is an integer selected from 1, 2, 3, 4, 5 and 6, preferably 2.

10. A modified solid support according to claim 1 having the following formula: 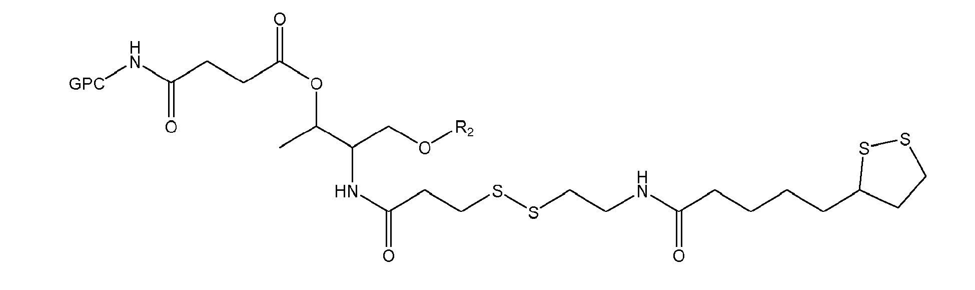 wherein R₂ is selected from H and a hydroxyl protecting group.

11. A modified solid support according to claim 10 having the following formula: 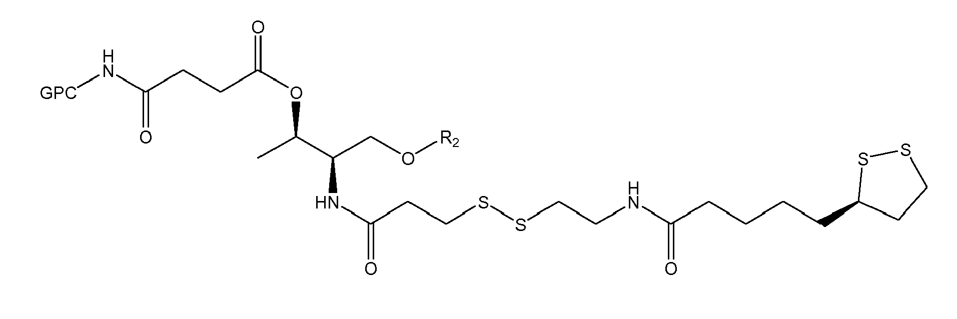 wherein R₂ is selected from H and a hydroxyl protecting group; or an enantiomer, diastereoisomer or mixtures thereof.

12. A method for preparing the modified solid support as defined in any of claims 1 to 11, comprising:
(i) reacting a compound of formula (II): 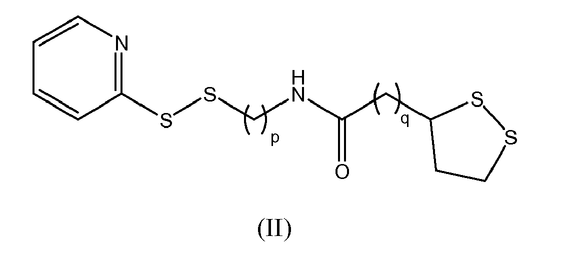 with a thiol of formula (III): 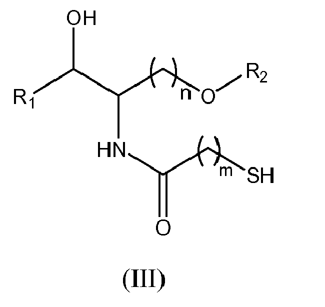 to afford a compound of formula (IV): 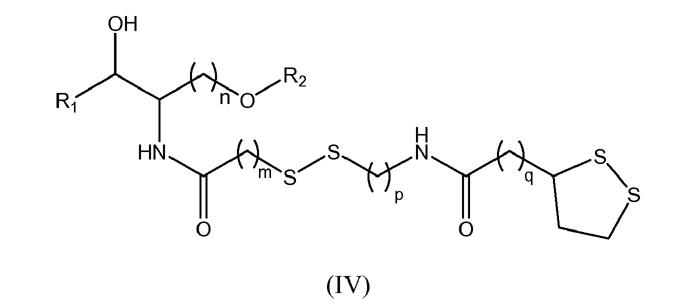
(ii) when R₂ is H, protecting said primary hydroxyl group with a hydroxyl protecting group,
(iii) reacting a compound of formula (IV), wherein R₂ is a hydroxyl protecting group, with a compound of formula (V): 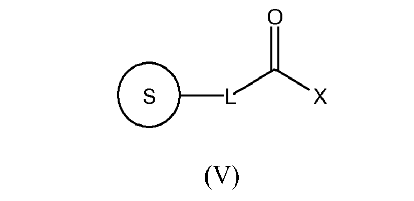
(iv) optionally, deprotecting the hydroxyl protecting group,
wherein:
X is selected from OH, Cl and Br;
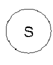 represents a solid support;
L represents a divalent linker;
R₁ is selected from C₁-C₆ alkyl, aryl and heteroaryl;
R₂ is selected from H and a hydroxyl protecting group;
n, m and p are independently an integer selected from 1, 2 and 3; and
q is an integer selected from 2, 3, 4, 5 and 6.

13. A method for the synthesis of oligonucleotides comprising the use of a modified solid support as defined in any one of claims 1 to 11.

14. A compound of formula (IV): 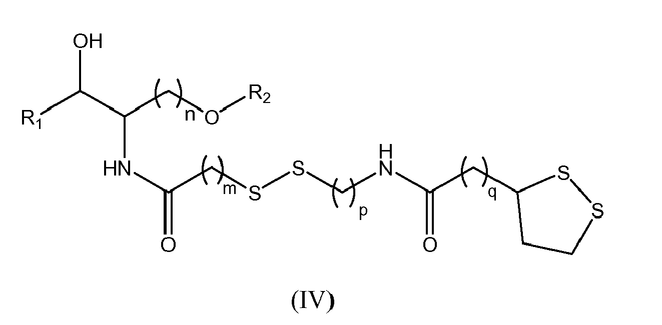 wherein:
R₁ is selected from C₁-C₆ alkyl, aryl and heteroaryl;
R₂ is selected from H and a hydroxyl protecting group;
n, m and p are independently an integer selected from 1, 2 and 3; and
q is an integer selected from 2, 3, 4, 5 and 6.

15. A compound according to claim 14 having the formula: 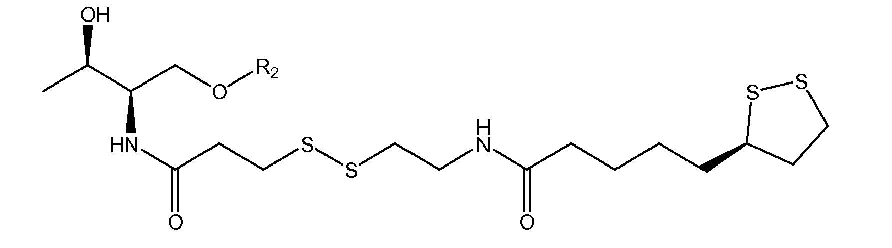 wherein R₂ is selected from H and a hydroxyl protecting group;
or an enantiomer, diastereoisomer or mixtures thereof.


SEQUENCE LISTING

<110>  FUNDACIÓN IMDEA NANOCIENCIA

<120>  MODIFIED SOLID SUPPORT FOR THE SYNTHESIS OF OLIGONUCLEOTIDES

<130>  P9482EP00

<160>  3

<170>  PatentIn version 3.5

<210>  1
<211>  26
<212>  DNA
<213>  Artificial Sequence

<220>
<223>  ON sequence as in Oligo-1

<400>  1
tcgaagtatt ccgcgtacgt tttttt                                          26


<210>  2
<211>  20
<212>  DNA
<213>  Artificial Sequence

<220>
<223>  Oligonucleotide sequence as in Oligo-2

<400>  2
cgtacgcgga atacttcgat                                                 20


<210>  3
<211>  10
<212>  DNA
<213>  Artificial Sequence

<220>
<223>  Oligonucleotide sequence as in Oligo-3 and Oligo-4

<400>  3
tttttttttt                                                            10